# EUROPEAN PATENT APPLICATION

(11) **EP 4 679 077 A1**
(43) Date of publication of application: **14.01.2026**
(21) Application number: 23926079.7
(22) Date of filing: 13.12.2023
(51) Int. Cl.: G01N 27/447

(54) **OLIGOSACCHARIDE CHAIN-BASED DETECTION REAGENT FOR DETECTING INTESTINAL CANCER, PREPARATION METHOD, AND USE**

(30) Priority: 03.03.2023 CN 202310211925
(71) Applicant: Xiansida Nanjing Biotechnology Co., Ltd., Nanjing, Jiangsu 211800 (CN); Jiangsu Xiansida Biotechnology Co., Ltd., Taizhou, Jiangsu 225312 (CN)
(72) Inventor: CHEN, Cuiying, Nanjing, Jiangsu 211800 (CN); FU, Tingting, Nanjing, Jiangsu 211800 (CN); ZHANG, Junli, Nanjing, Jiangsu 211800 (CN); XU, Lei, Nanjing, Jiangsu 211800 (CN); LI, Weiquan, Nanjing, Jiangsu 211800 (CN)
(74) Representative: Proi World Intellectual Property GmbH
(86) International application number: PCT/CN2023/138510
(87) International publication number: WO 2024/183385

(57) **Abstract**

An oligosaccharide chain-based detection reagent for detecting intestinal cancer, a preparation method therefor, and use thereof. The detection reagent is prepared by mixing the following reagents: reagent A, which is prepared by adding SDS with a mass concentration of 1-5% to an ammonium bicarbonate solution with a concentration of 10 mM and a pH of 8.3; reagent B, which is prepared by mixing 0.05-10 unit/10 µl of glucosaminidase, NP-40 with a mass concentration of 10%, and an ammonium bicarbonate solution with a concentration of 10 mM and a pH of 8.3, the pH value of the mixed solution being 5-9; reagent C, which is an organic reducing agent with a concentration of 0.02 mM-1 M prepared by dissolving 8-aminopyrene-1,3,6-trisulfonic acid in DMSO; and reagent D, which is a stop solution. A natural oligosaccharide N-glycome profile in a serum is measured by means of the reagent, then peak values are quantified, and statistical analysis is performed, so that a method for establishing a serum natural oligosaccharide N-glycome profile model for intestinal cancer is provided, and intestinal cancer is detected by means of determining changes in the natural glycosylation modification of a protein in a physiological and pathological state.

## Description

### Technical Field

The present invention belongs to the field of biopharmaceutical technology, and relates to a method for detecting prostate cancer, specifically to a prostate cancer detection method based on a G-Test-specific fingerprint profile derived from serum oligosaccharide chains.

### Background technology

Prostate cancer is one of the common malignant tumors in the genitourinary system among elderly males. Globally, its incidence and mortality rates rank second and fifth respectively among male malignancies, while in China, these figures occupy the sixth and seventh positions among male neoplasms. The survival period of prostate cancer patients is closely associated with the clinical stage of malignancy at diagnosis. Due to its insidious onset and slow progression, the majority of initially diagnosed cases present with intermediate to advanced clinical stages, with merely 30% being clinically localized cases. This pathological characteristic contributes to the overall poor prognosis of prostate cancer patients in China. Consequently, early screening and diagnosis constitute a prerequisite for prolonging the survival period of prostate cancer patients.

Clinically employed screening modalities encompass detection of serum biomarker prostate-specific antigen (PSA), digital rectal examination (DRE), and imaging examinations. However, each technical approach exhibits inherent limitations. Although PSA screening has significantly enhanced prostate cancer detection rates, 23% to 42% of screening-detected cases result in overtreatment, imposing increased socioeconomic healthcare burdens while inflicting unnecessary physical distress and iatrogenic harm on patients. DRE, while the most cost-effective modality, demonstrates diagnostic accuracy of merely 30% to 50%, with performance heavily dependent on operator expertise. Imaging techniques exhibit low sensitivity for early-stage prostate cancer detection. Given the limitations inherent to these diagnostic methods, there is an urgent need to develop novel non-invasive methodologies for auxiliary prostate cancer diagnosis with improved sensitivity and specificity.

Protein glycosylation, the most prevalent post-translational modification, involves the enzymatic transfer of carbohydrate moieties to asparagine (Asn) amine groups via N-glycan or to serine/threonine hydroxyl oxygen atoms via O-glycan, mediated by glycosyltransferases. This process critically regulates protein functionality. The majority of glycoproteins are secretory proteins, ubiquitously distributed across cellular membranes, extracellular matrices, plasma, and mucous secretions. N-glycan chains on glycoproteins undergo structural maturation and functional diversification to modulate protein conformation, stability, and catalytic activity. Consequently, glycan structures play indispensable roles in maintaining organismal biological homeostasis, endowing glycoproteins with diverse biofunctional properties. Elucidating glycan structural alterations is therefore pivotal for deciphering molecular mechanisms underlying pathological processes such as inflammatory responses and tumor cell invasion/metastasis.

Aberrant alterations in protein N-glycan structures have been identified across multiple tumor types, with terminal sialic acid modifications representing a critical subset of these changes. Sialic acids, a family of negatively charged 9-carbon monosaccharides ubiquitously distributed in biological systems, predominantly occupy terminal positions on glycan chains. These residues are enzymatically linked via α-2,3 or α-2,6 glycosidic linkages to galactose or N-acetylgalactosamine moieties under sialidase catalysis, forming polysialic acid chains. Research has demonstrated that terminal sialic acid residues on glycoproteins modulate essential biological processes including cellular recognition, molecular interactions, viral infectivity, immune responses, and signal transduction, with sialylation patterns exhibiting tissue- and cell-specific expression profiles. Substantial research evidence demonstrates that aberrant sialylation contributes to the pathogenesis of multiple diseases, including the onset of infectious pathologies and the progression of neoplastic infiltration and metastasis. Consequently, detecting alterations in sialic acid-associated oligosaccharide chains holds potential clinical validity for auxiliary diagnostic applications in prostate cancer management.

### Invention content

To address the current limitations in clinical prostate cancer detection, including overtreatment in PSA-screened patients, low accuracy of digital rectal examination (DRE), and limited sensitivity of imaging diagnostics, the present invention provides a prostate cancer detection reagent. This reagent analyzes the natural oligosaccharide N-glycome profile in serum by quantifying glycan peaks, performing statistical analysis, and establishing a serum-based N-glycome profile model for prostate cancer. The method detects prostate cancer by measuring alterations in natural glycosylation modifications of proteins under pathophysiological conditions.

### The technical solution adopted by the present invention is as follows:

A prostate cancer detection reagent based on oligosaccharide chain analysis, characterized by comprising the following reagents:
Reagent A: Prepared by adding 1-5%(w/v) SDS to a 10 mM ammonium bicarbonate solution (pH 8.3);
Reagent B: Formulated by mixing 0.05-10 units/10 µL of glycosaminidase, 10% (w/v) NP-40, and a 10 mM ammonium bicarbonate solution (pH 8.3), with the final mixed solution maintaining a pH range of 5-9;
Reagent C: An organic reductant prepared by dissolving 8-aminopyrene-1,3,6-trisulfonic acid (APTS) in DMSO at a concentration of 0.02 mM to 1 M;
Reagent D: Termination solution.

In some embodiments, Reagent A, Reagent B, and Reagent C are provided in a volumetric ratio of 1:1:1.

In some embodiments, each of Reagent A, Reagent B, and Reagent C is present at 5 µL.

In some embodiments, Reagent D comprises ultrapure water.

A method for preparing an oligosaccharide chain-based detection reagent for prostate cancer diagnosis, comprising the steps of:

### Step 1: Preparation of Oligosaccharide Chains

Add 5 µL of Reagent A to 5 µL of inactivated serum sample for denaturation. After cooling to room temperature, add 5µL of Reagent B and incubate at 37°C for 3 hours (h) in a controlled thermal cycler, followed by vacuum drying.

### Step 2: Oligosaccharide Chain Labeling

Add 5µL of Reagent C to the dried sample obtained from Step 1. Incubate the mixture at 60°C for 1 hour (h) in a thermal-controlled hybridization oven to perform fluorescent labeling. Subsequently, add 100 µL of Reagent D to terminate the labeling reaction.

### Step 3: Separation and Analysis of Oligosaccharide Chains

Transfer 10µL of the labeled oligosaccharide solution to an analytical instrument for N-linked oligosaccharide separation assay. Perform the analysis to obtain the native oligosaccharide N-glycome profile.

### Step 4: Data Processing and Analysis

Quantification of N-Glycome Profile Peaks: Calculate the relative abundance of each peak by dividing its peak height value by the sum of all peak heights in the chromatogram.

Use of a composition in the preparation of an oligosaccharide chain-based prostate cancer detection reagent, characterized in that: said composition comprises serum-derived glycans G4S4, G3S3, G2S2, G2S2F, G2S1, G2S1F, G1F, and G2F2, wherein prostate cancer detection is achieved through the ratio parameter calculated as G1S1F/G3S3 + G1S1F/G2S2F.

The G1F is an isomer.

The present invention provides a method for establishing a serum natural oligosaccharide N-glycan profile model for prostate cancer, which comprises determining a G-Test-specific fingerprint profile of serum natural oligosaccharide chains and conducting statistical analysis.

### Material and method:

**1. Test samples:** Serum samples from prostate cancer patients and healthy donors.
**2. Experimental apparatus:** Capillary electrophoresis analyzer, PCR thermocycler, and centrifuge.

### 3. Reagent preparation:

(1) Reagent A: Prepared by adding 1~5% (w/v) sodium dodecyl sulfate (SDS) to 10 mM ammonium bicarbonate solution (pH 8.3).
(2) Reagent B: Formulated by mixing 0.05 units/10µL glycosidase, 10% (w/v) NP-40 detergent, and 10 mM ammonium bicarbonate buffer (pH 8.3), with the final solution adjusted to pH 5~9.
(3) Reagent C: Comprising 0.02mM ~1M 8-aminopyrene-1,3,6-trisulfonic acid (APTS) dissolved in dimethyl sulfoxide (DMSO) as an organic reductant.
(4) Reagent D: Ultrapure water.

### 4. N-Glycome Profiling:

(1) Oligosaccharide Chain Preparation
   To 5µL of heat-inactivated serum sample, 5µL of Reagent A was added for denaturation. Following cooling to ambient temperature, 5µL of Reagent B was introduced and incubated at 37°C for 3 hours, after which the mixture was dried under vacuum.
(2) Oligosaccharide Chain Labeling
   1) The dried sample was reconstituted with 5µL of Reagent C and reacted at 60°C for 1 hour to achieve fluorophore conjugation.
   2) The labeling reaction was quenched by adding 100µL of Reagent D.
(3) Oligosaccharide Chain Separation and Analysis
   10µL of the labeled oligosaccharide solution was analyzed via N-oligosaccharide chain separation using an ABI 3500dx Analyzer, thereby obtaining native N-glycan profiles.
(4) Data Processing and Analysis
   1) Quantification of N-glycan Profile Peaks: The relative abundance of each glycan species was calculated by dividing the peak height of individual peaks by the sum of all peak heights.
   2) Comparative Analysis of Prostate Cancer Patients vs. Healthy Controls: Statistical comparison of N-glycan profiles between prostate cancer patients and healthy individuals was performed. The relative content of each peak was quantitatively determined by dividing the individual peak height value by the sum of all peak heights, thereby achieving peak quantification of the N-glycome profile. A comparative statistical analysis was subsequently performed on the quantified data of nine N-oligosaccharide peaks between the prostate cancer group and healthy control group in their respective N-glycome profiles. The N-glycome profile composition consisted of G4S4, G3S3, G2S2, G2S2F, G2S1, G2S1F, G1F (an isomer), and G2F2. Prostate cancer is detected using a composite value calculated as the sum of the ratios G1S1F/G3S3+G1S1F/G2S2F.

### Compared to prior techniques, the present invention demonstrates the following technical benefits:

(1) Quantitative analysis of G-Test fingerprint profile peaks through comparative evaluation of 186 prostate cancer patients and 198 healthy controls revealed statistically significant differences (p<0.05) in relative abundance of N-glycan compositions G3S3, G1S1F, and G2S2F between cohorts. The diagnostic model based on the combined ratio G1S1F/G3S3+G1S1F/G2S2F achieved an AUC of 0.854 in ROC curve analysis for prostate cancer discrimination. At an optimal cutoff value of 5.18, this composite biomarker demonstrated 81.60% sensitivity and 75.80% specificity, establishing serum N-glycan ratios G1S1F/G3S3+G1S1F/G2S2F as a clinically validated auxiliary diagnostic marker for prostate cancer.
(2) The G-Test methodology disclosed herein employs DNA sequencer-assisted fluorophore-assisted carbohydrate electrophoresis (DSA-FACE) for capillary microelectrophoretic separation of fluorescently labeled N-glycans from serum glycoproteins. This platform generates native oligosaccharide N-glycome profiles through quantitative fluorescence signal measurement, enabling precise characterization of sialylated oligosaccharide chain alterations under pathophysiological conditions. By establishing statistically validated correlations between disease states and sialic acid oligosaccharide chain structural variations, the invention constructs a predictive model of N-glycan composition ratios that aids in diagnosing prostate cancer status.
(3) The G-Test natural N-glycome profiling model established by the present invention enables routine, non-invasive testing for patients, assisting clinicians and individuals in timely monitoring of prostate cancer onset and progression. The detection technology exhibits the following advantages: high sensitivity, operational simplicity, minimal sample volume (5µL serum), high reproducibility, excellent stability, and high-throughput capability (96-well plate format), making it clinically applicable for widespread use.

### Figure Legends

Figure 1 illustrates serum-derived natural N-glycan profiles of healthy individuals and prostate cancer patients.
Figure 2 depicts a receiver operating characteristic (ROC) curve analysis of a prostate cancer diagnostic model based on the N-glycan composition ratio G1S1F/G3S3+G1S1F/G2S2F, derived from a total cohort of 384 samples (186 prostate cancer serum samples; 198 healthy controls), yielding an area under the curve (AUC) of 0.854.

### Mode of Carrying out the Invention

The present invention will now be further elucidated through embodiments and accompanying drawings. It should be noted that the following embodiments are provided solely for illustrative purposes and do not limit the scope of the present invention. Experimental methods not specifying particular conditions in the following embodiments were conducted under conventional laboratory conditions or according to manufacturer-recommended protocols.

The detection of prostate cancer status through analysis of serum-derived natural N-glycan profiles and subsequent statistical evaluation, as well as the materials and methods employed, are described in the following embodiments.

### Example 1

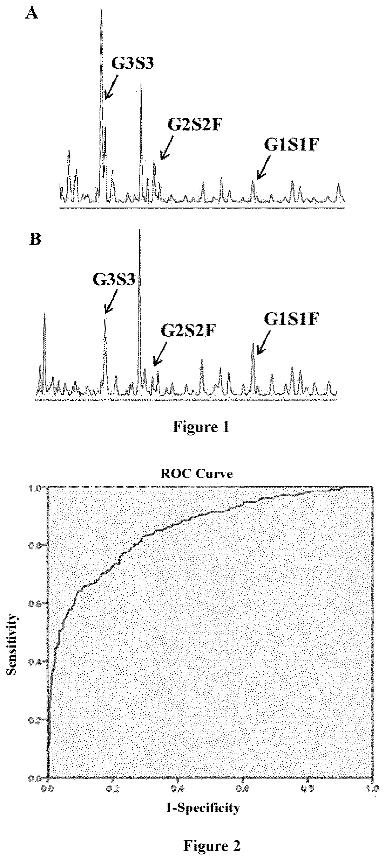

1, Test samples: Serum samples from prostate cancer (prostate cancer) patients and healthy donors.
2, Experimental apparatus: Capillary electrophoresis analyzer, PCR thermocycler, and centrifuge.
3, Reagent preparation:
   (1) Reagent A: Prepared by adding 2.5% (w/v) sodium dodecyl sulfate (SDS) to 10 mM ammonium bicarbonate solution.
   (2) Reagent B: Formulated by mixing 3.5 units/10µL glycosidase, 10% (w/v) NP-40 detergent, and 10 mM ammonium bicarbonate buffer (pH 8.3), with the final solution adjusted to pH 7.0.
   (3) Reagent C: Comprising 10 mM 8-aminopyrene-1,3,6-trisulfonic acid (APTS) dissolved in dimethyl sulfoxide (DMSO) as an organic reductant.
   (4) Reagent D: Ultrapure water.
4. N-Glycome Profiling:
   (1) Oligosaccharide Chain Preparation
      To 5µL of heat-inactivated serum sample, 5µL of Reagent A was added for denaturation. Following cooling to ambient temperature, 5µL of Reagent B was introduced and incubated at 37°C for 3 hours, after which the mixture was dried under vacuum.
   (2) Oligosaccharide Chain Labeling
      1) The dried sample was reconstituted with 5µL of Reagent C and reacted at 60°C for 1 hour to achieve fluorophore conjugation.
      2)The labeling reaction was quenched by adding 100µL of Reagent D.
   (3) Oligosaccharide Chain Separation and Analysis
      10µL of the labeled oligosaccharide solution was analyzed via N-oligosaccharide chain separation using an ABI 3500dx Analyzer, thereby obtaining native N-glycan profiles.
   (4) Data Processing and Analysis
      1) Quantification of N-glycan Profile Peaks: The relative abundance of each glycan species was calculated by dividing the peak height of individual peaks by the sum of all peak heights.
      2) Comparative Analysis of Prostate Cancer Patients vs. Healthy Controls: Statistical comparison of N-glycan profiles between prostate cancer patients and healthy individuals was performed. As shown in Figure 1, human serum N-glycan profiles exhibit approximately 9 distinct N-oligosaccharide peaks. Differential electrophoretic mobility, attributable to variations in charge-to-mass ratios and molecular sizes, enables resolution of individual oligosaccharides as discrete peaks, with peak heights corresponding to their relative abundances. In **Figure 1A****:** N-glycan profile of healthy donor serum. In **Figure 1B****:** N-glycan profile of prostate cancer patient serum. The N-glycan compositional repertoire comprises G4S4, G3S3, G2S2, G2S2F, G2S1, G2S1F, G1F (an isomer), and G2F2. Diagnostic evaluation of prostate cancer status is facilitated by calculating the composite ratio: Diagnostic Index = G1S1F/G3S3+G1S1F/G2S2F.

The diagnostic model established based on the composite biomarker ratio G1S1F/G3S3+G1S1F/G2S2F achieved an area under the receiver operating characteristic (ROC) curve (AUC) of 0.854 (Figure 2) for discriminating prostate cancer patients. At a predetermined cutoff value of 5.18, this ratio demonstrated 81.60% sensitivity and 75.80% specificity for prostate cancer detection, thereby validating the serum N-glycan composition ratio G1S1F/G3S3+G1S1F/G2S2F as an auxiliary diagnostic biomarker for prostate cancer.

### Example 2

1, Test samples: Serum samples from prostate cancer (prostate cancer) patients and healthy donors.
2, Experimental apparatus: Capillary electrophoresis analyzer, PCR thermocycler, and centrifuge.
3, Reagent preparation:
   (1) Reagent A: Prepared by adding 1% (w/v) sodium dodecyl sulfate (SDS) to 10 mM ammonium bicarbonate solution.
   (2) Reagent B: Formulated by mixing 0.05 units/10 µL glycosidase, 10% (w/v) NP-40 detergent, and 10 mM ammonium bicarbonate buffer (pH 8.3), with the final solution adjusted to pH 5.0.
   (3) Reagent C: Comprising 0.02 mM 8-aminopyrene-1,3,6-trisulfonic acid (APTS) dissolved in dimethyl sulfoxide (DMSO) as an organic reductant.
   (4) Reagent D: Ultrapure water.
4, The detection of N-glycan profiling is the same as that in Example 1.

### Example 3

1, Test samples: Serum samples from prostate cancer patients and healthy donors.
2, Experimental apparatus: Capillary electrophoresis analyzer, PCR thermocycler, and centrifuge.
3, Reagent preparation:
   (1) Reagent A: Prepared by adding 5% (w/v) sodium dodecyl sulfate (SDS) to 10 mM ammonium bicarbonate solution.
   (2) Reagent B: Formulated by mixing 10 units/10µL glycosidase, 10% (w/v) NP-40 detergent, and 10 mM ammonium bicarbonate buffer (pH 8.3), with the final solution adjusted to pH 9.0.
   (3) Reagent C: Comprising 1M 8-aminopyrene-1,3,6-trisulfonic acid (APTS) dissolved in dimethyl sulfoxide (DMSO) as an organic reductant.
   (4) Reagent D: Ultrapure water.
4. The detection of N-glycan profiling is the same as that in Example 1.

Compared to prior techniques, the present detection method achieves technical advancement by correlating pathological alterations in sialylated oligosaccharide chains under pathophysiological conditions with disease states, thereby enabling construction of a predictive model based on N-glycan composition ratios for auxiliary determination of prostate cancer status. The G-Test natural N-glycome profiling model established through this invention facilitates routine, non-invasive testing across patient populations, allowing clinicians and patients to monitor prostate cancer initiation and progression in a timely manner. This methodology demonstrates immediate clinical translation potential for large-scale implementation.

The foregoing specific embodiments, described in conjunction with the accompanying drawings, further elucidate the objectives, technical solutions, and advantageous effects of the present invention. It shall be understood that these embodiments are provided solely to illustrate the invention and shall not be construed as limiting its protective scope. Those skilled in the art will recognize that any modifications, equivalent substitutions, improvements, and the like made within the spirit and principles of the present invention-without requiring inventive effort-shall fall within the scope of protection defined by the appended claims.

## Claims

1. A prostate cancer detection reagent based on oligosaccharide chain analysis,
**characterized by** comprising the following reagents:
Reagent A: Prepared by adding 1-5% (w/v) SDS to a 10 mM ammonium bicarbonate solution (pH 8.3);
Reagent B: Formulated by mixing 0.05-10 units/10µL of glycosaminidase, 10% (w/v) NP-40, and a 10 mM ammonium bicarbonate solution (pH 8.3), with the final mixed solution maintaining a pH range of 5-9;
Reagent C: An organic reductant prepared by dissolving 8-aminopyrene-1,3,6-trisulfonic acid (APTS) in DMSO at a concentration of 0.02 mM to 1 M;
Reagent D: Termination solution.

2. The prostate cancer detection reagent based on oligosaccharide chain analysis according to claim 1, **characterized in that** the volume ratio of Reagent A, Reagent B, and Reagent C is 1:1:1.

3. The prostate cancer detection reagent based on oligosaccharide chain analysis according to claim 2, **characterized in that** each of Reagent A, Reagent B, and Reagent C has a volume of 5 µL.

4. The prostate cancer detection reagent based on oligosaccharide chain analysis according to claim 1, **characterized in that** Reagent D is ultrapure water.

5. A preparation method for the prostate cancer detection reagent based on oligosaccharide chain analysis according to claim 1, **characterized by** comprising the following steps:
**Step 1: Preparation of Oligosaccharide Chains**
Add 5µL of **Reagent A** to 5µL of **inactivated serum sample** for denaturation. After cooling to room temperature, add 5µL of **Reagent B** and incubate at 37°C for 3 hours (h) in a controlled thermal cycler, followed by vacuum drying.
**Step 2: Oligosaccharide Chain Labeling**
Add 5µL of Reagent C to the dried sample obtained from Step 1. Incubate the mixture at 60°C for 1 hour (h) in a thermal-controlled hybridization oven to perform fluorescent labeling. Subsequently, add 100µL of Reagent D to terminate the labeling reaction.
**Step 3: Separation and Analysis of Oligosaccharide Chains**
Transfer 10µL of the labeled oligosaccharide solution to an analytical instrument for N-linked oligosaccharide separation assay. Perform the analysis to obtain the native oligosaccharide N-glycan profile.
**Step 4: Data Processing and Analysis**
Quantification of N-Glycome Profile Peaks: Calculate the relative abundance of each peak by dividing its peak height value by the sum of all peak heights in the chromatogram.

6. Use of a composition in the preparation of an oligosaccharide chain-based prostate cancer detection reagent, **characterized in that**: said composition comprises serum-derived glycans G4S4, G3S3, G2S2, G2S2F, G2S1, G2S1F, G1F, and G2F2, wherein prostate cancer detection is achieved through the ratio parameter calculated as G1S1F/G3S3 + G1S1F/G2S2F.

7. The use of the composition in preparing an oligosaccharide chain-based prostate cancer detection reagent according to claim 6, **characterized in that**: said G1F exists as structural isomers.
